# EUROPEAN PATENT SPECIFICATION

(11) **EP 4 306 644 B1**
(45) Date of publication and mention of the grant of the patent: **29.07.2026**
(21) Application number: 21930458.1
(22) Date of filing: 30.04.2021
(51) Int. Cl.: C12N 15/77, C12N 9/88, C12P 13/08

(54) **CORYNEBACTERIUM GLUTAMICUM VARIANT HAVING IMPROVED L-LYSINE PRODUCTION ABILITY, AND METHOD FOR PRODUCING L-LYSINE BY USING SAME**
CORYNEBACTERIUM GLUTAMICUM-VARIANTE MIT VERBESSERTER L-LYSIN-PRODUKTIONSFÄHIGKEIT UND VERFAHREN ZUR HERSTELLUNG VON L-LYSIN DAMIT
VARIANT DE CORYNEBACTERIUM GLUTAMICUM À CAPACITÉ AMÉLIORÉE DE PRODUCTION DE L-LYSINE ET PROCÉDÉ DE PRODUCTION DE L-LYSINE FAISANT APPEL À CELUI-CI

(30) Priority: 09.03.2021 KR 20210030960; 27.04.2021 KR 20210054313
(43) Date of publication of application: 17.01.2024
(73) Proprietor: CJ Cheiljedang Corporation, Seoul 04560 (KR)
(72) Inventor: RYU, Mi, Gyeonggi-do 17384 (KR); MOON, Min Woo, Daejeon 34150 (KR); HONG, In Pyo, Gyeonggi-do 17384 (KR); PARK, Seok Hyun, Gyeonggi-do 12258 (KR); PARK, Joon Hyun, Gyeonggi-do 13523 (KR)
(74) Representative: Regimbeau
(86) International application number: PCT/KR2021/005509
(87) International publication number: WO 2022/191358

(56) References cited:
- EP-A1- 3 170 889
- WO-A2-01/49854
- WO-A2-2018/226964
- KR-A- 20080 025 355
- KR-A- 20130 130 377
- US-A1- 2020 347 419
- CHERAGHI, S.: "Improved production of l-lysine by over-expression of meso-diaminopimelate decarboxylase enzyme of corynebacterium glutamicum in escherichia coli", PAKISTAN JOURNAL OF BIOLOGICAL SCIENCES, INET: ASIAN NETWORK FOR SCIENTIFIC INFORMATION, vol. 13, no. 10, 1 January 2010 (2010-01-01), INET: Asian Network for Scientific Information , pages 504 - 508, XP055967888, ISSN: 1812-5735
- DATABASE Nucleotide 27 December 2017 (2017-12-27), ANONYMOUS : "Corynebacterium glutamicum strain HA chromosome, complete genome. ", XP055968542, retrieved from NCBI Database accession no. CP025534.1

## Description

### Technical Field

The present disclosure relates to a *Corynebacterium glutamicum* variant with improved L-lysine producing ability and a method of producing L-lysine using the same.

### Background Art

L-Lysine is an essential amino acid that is not synthesized in the human or animal body and must be supplied from the outside, and is generally produced through fermentation using microorganisms such as bacteria or yeast. In L-lysine production, naturally obtained wild-type strains or variants modified to have enhanced L-lysine producing ability thereof may be used. Recently, in order to improve the production efficiency of L-lysine, various recombinant strains or variants with excellent L-lysine producing ability and methods of producing L-lysine using the same have been developed by applying a genetic recombination technology to microorganisms such as *Escherichia coli* and *Corynebacterium, etc.,* which are widely used in the production of L-amino acids and other useful substances.

According to Korean Patent Nos. 10-0838038 and 10-2139806, nucleotide sequences of genes encoding proteins including enzymes related to L-lysine production or amino acid sequences thereof are modified to increase expression of the genes or to remove unnecessary genes and thereby improve the L-lysine producing ability. In addition, Korean Patent Publication No. 10-2020-0026881 discloses a method of replacing the existing promoter of a gene with a promoter with strong activity in order to increase expression of the gene encoding the enzyme involved in L-lysine production.

Patent document WO0149854A2 discloses a promoter operably linked to LysA gene encoding diaminopimelate decarboxylase and uses in the production of L-Lysine thereof.

As described, a variety of methods to increase the L-lysine producing ability are being developed. Nevertheless, since there are dozens of types of proteins such as enzymes, transcription factors, transport proteins, *etc.* which are directly or indirectly involved in the L-lysine production, it is necessary to conduct many studies on whether or not the L-lysine producing ability is increased according to changes in the activities of these proteins.

### [Prior Art Documents]

### [Patent Documents]

Korean Patent No 10-0838038
Korean Patent No. 10-2139806
Korean Publication Patent No. 10-2020-0026881

### Disclosure

### Technical Problem

An object of the present disclosure is to provide a *Corynebacterium glutamicum* variant with improved L-lysine producing ability.

Further, another object of the present disclosure is to provide a method of producing L-lysine using the variant.

### Technical Solution

The present inventors have studied to develop a novel variant with improved L-lysine producing ability using a *Corynebacterium glutamicum* strain, and as a result, they found that L-lysine production is increased by substituting a nucleotide sequence at a specific position in a promoter of lysA gene encoding diaminopimelate decarboxylase, which acts in the last step of the L-lysine biosynthetic pathway, thereby completing the present disclosure.

The present invention is defined by the claims.

An aspect of the present disclosure provides a *Corynebacterium glutamicum* variant with improved L-lysine producing ability by enhancing the activity of diaminopimelate decarboxylase.

As used herein, the "diaminopimelate decarboxylase" refers to an enzyme that catalyzes a reaction of producing carbon dioxide and L-lysine by cleaving carbon bonds of meso-diaminoheptanedioate (mDAP) in the last step of the L-lysine biosynthetic pathway.

According to a specific embodiment of the present disclosure, the diaminopimelate decarboxylase may be derived from a strain of the genus *Corynebacterium.* Specifically, the strain of the genus *Corynebacterium* may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens,* but is not limited thereto

As used herein, "enhancing the activity" means that expression levels of genes encoding proteins such as target enzymes, transcription factors, transport proteins, *etc.* are increased by newly introducing or enhancing the genes, as compared to those of a wild-type strain or a strain before modification. Such enhancement of the activity also includes the case where activity of the protein itself is increased through substitution, insertion, or deletion of the nucleotide encoding the gene, or a combination thereof, as compared to activity of the protein originally possessed by a microorganism, and the case where the overall enzyme activity in cells is higher than that of the wild-type strain or the strain before modification, due to increased expression or increased translation of the genes encoding the same, and a combination thereof

According to a specific embodiment of the present disclosure, enhancement of the activity of diaminopimelate decarboxylase may induce a site-specific mutation in a promoter of a gene encoding diaminopimelate decarboxylase.

According to a specific embodiment of the present disclosure, the promoter of the gene encoding diaminopimelate decarboxylase may be represented by a nucleotide sequence of SEQ ID NO: 1.

As used herein, the "promoter" refers to a specific region of DNA that regulates gene transcription by including the binding site for RNA polymerase that initiates mRNA transcription of a gene of interest, and is generally located upstream of the transcription start site. The promoter in prokaryotes is defined as a region near the transcription start site where RNA polymerase binds, and generally consists of two short nucleotide sequences at -10 and -35 base-pair regions upstream from the transcription start site. In the present disclosure, the promoter mutation is that the promoter is improved to have high activity, as compared to a wild-type promoter, and may increase the expression of genes located downstream by inducing mutations in the promoter region located upstream of the transcription start site.

According to a specific embodiment of the present disclosure, the enhancement of the activity of diaminopimelate decarboxylase may be substitution of one or more bases at -25 to -10 regions upstream from the transcription start site in the promoter sequence of the gene encoding diaminopimelate decarboxylase.

More specifically, the promoter mutation of the present disclosure may be consecutive or non-consecutive substitution of one or more bases at -25 to -10 regions, preferably, consecutive or non-consecutive substitution of one, two, three, four, or five bases at -20 to -15 regions, at -19 to -16 regions, or at -18 and -17 regions.

According to one exemplary embodiment of the present disclosure, lysA gene encoding diaminopimelate decarboxylase forms an operon together with argS gene encoding arginyl-tRNA synthetase, and argS-lysA operon is regulated by a single promoter. Therefore, CG which is a nucleotide sequence at -17 and -18 regions of the promoter sequence of argS-lysA operon of the *Corynebacterium glutamicum* strain is substituted with CT to obtain a *Corynebacterium glutamicum* variant having a new promoter sequence of lysA gene. Such a *Corynebacterium glutamicum* variant may include the mutated promoter sequence of lysA gene, which is represented by a nucleotide sequence of SEQ ID NO: 2.

Further, according to one exemplary embodiment of the present disclosure, CG which is a nucleotide sequence at -17 and -18 regions of the promoter sequence of argS-lysA operon of the *Corynebacterium glutamicum* strain is substituted with GA to obtain a *Corynebacterium glutamicum* variant having a new promoter sequence of lysA gene. Such a *Corynebacterium glutamicum* variant may include the mutated promoter sequence of lysA gene, which is represented by a nucleotide sequence of SEQ ID NO: 3.

According to one exemplary embodiment of the present disclosure, CG which is a nucleotide sequence at -17 and -18 regions of the promoter sequence of argS-lysA operon of the *Corynebacterium glutamicum* strain is substituted with GT to obtain a *Corynebacterium glutamicum* variant having a new promoter sequence of lysA gene. Such a *Corynebacterium glutamicum* variant may include the mutated promoter sequence of lysA gene, which is represented by a nucleotide sequence of SEQ ID NO: 4.

As used herein, the "improved production ability" means increased L-lysine productivity, as compared to that of a parent strain. The parent strain refers to a wild-type or variant strain that is a subject of mutation, and includes a subject that is directly mutated or transformed with a recombinant vector, *etc.* In the present disclosure, the parent strain may be a wild-type *Corynebacterium glutamicum* strain or a strain mutated from the wild-type.

According to a specific embodiment of the present disclosure, the parent strain is a variant in which mutations are induced in the sequences of genes (e.g., lysC, zwf, and hom genes) involved in the lysine production, and may be a *Corynebacterium glutamicum* strain (hereinafter referred to as "Corynebacterium glutamicum DS1 strain") deposited at the Korean Culture Center of Microorganisms on April 2, 2021, with Accession No. KCCM12969P.

According to one exemplary embodiment of the present disclosure, the *Corynebacterium glutamicum* variant with improved L-lysine producing ability may include the promoter mutation of lysA gene encoding diaminopimelate decarboxylase, thereby exhibiting the increased L-lysine producing ability, as compared to the parent strain, and in particular, may exhibit 2% or more, specifically 2% to 40%, and more specifically 3% to 30% increase in the L-lysine production, as compared to the parent strain, thereby producing 60 g to 80 g of L-lysine, preferably 65 g to 75 g of L-lysine per 1 L of the culture of the strain.

The *Corynebacterium glutamicum* variant according to a specific embodiment of the present disclosure may be achieved through a recombinant vector including a variant in which part of the promoter sequence of the gene encoding diaminopimelate decarboxylase in the parent strain is substituted.

As used herein, the "part" means not all of a nucleotide sequence or polynucleotide sequence, and may be 1 to 300, preferably 1 to 100, and more preferably 1 to 50, but is not limited thereto.

As used herein, the "variant" refers to a promoter variant in which one or more bases at -25 to -10 regions in the promoter sequence of the diaminopimelate decarboxylase gene involved in the L-lysine biosynthesis are substituted.

According to a specific embodiment of the present disclosure, variants, each in which the nucleotide sequence at -17 and -18 regions in the promoter sequence of the diaminopimelate decarboxylase gene is substituted with CT, GA, or GT, may have the nucleotide sequence of SEQ ID NO: 2, SEQ ID NO: 3, or SEQ ID NO: 4, respectively.

As used herein, the "vector" is an expression vector capable of expressing a target protein in a suitable host cell, and refers to a gene construct including essential regulatory elements which are operably linked so that a gene insert is expressed. Here, "operably linked" means that a gene requiring expression and a regulatory sequence thereof are functionally linked to each other to induce gene expression, and the "regulatory elements" include a promoter for performing transcription, any operator sequence for controlling the transcription, a sequence encoding a suitable mRNA ribosome binding site, and a sequence controlling termination of transcription and translation. Such a vector may include a plasmid vector, a cosmid vector, a bacteriophage vector, a viral vector, *etc.,* but is not limited thereto.

As used herein, the "recombinant vector" is transformed into a suitable host cell and then replicated independently of the genome of the host cell, or may be integrated into the genome itself. In this regard, the "suitable host cell", where the vector is replicable, may include the origin of replication which is a particular nucleotide sequence at which replication is initiated.

For the transformation, an appropriate technology of introducing the vector is selected depending on the host cell to express the target gene in the host cell. For example, introduction of the vector may be performed by electroporation, heatshock, calcium phosphate (CaPO₄) precipitation, calcium chloride (CaCl₂) precipitation, microinjection, a polyethylene glycol (PEG) method, a DEAE-dextran method, a cationic liposome method, a lithium acetate-DMSO method, or combinations thereof. As long as the transformed gene may be expressed within the host cell, it may be included without limitation, regardless of whether or not the gene is inserted into the chromosome of the host cell or located outside the chromosome.

The host cells include cells transfected, transformed, or infected with the recombinant vector or polynucleotide of the present disclosure *in vivo* or *in vitro.* Host cells including the recombinant vector of the present disclosure are recombinant host cells, recombinant cells, or recombinant microorganisms.

Further, the recombinant vector of the present disclosure may include a selection marker, which is for selecting a transformant (host cell) transformed with the vector. In a medium treated with the selection marker, only cells expressing the selection marker may survive, and thus transformed cells may be selected. The selection marker may be represented by kanamycin, streptomycin, chloramphenicol, *etc.,* but is not limited thereto.

The genes inserted into the recombinant vector for transformation of the present disclosure may be introduced into host cells such as microorganisms of the genus *Corynebacterium* due to homologous recombination crossover.

According to a specific embodiment of the present disclosure, the host cell may be a strain of the genus *Corynebacterium,* for example, *Corynebacterium glutamicum* DS1 strain.

Further, another aspect of the present disclosure provides a method of producing L-lysine, the method including the steps of a) culturing the *Corynebacterium glutamicum* variant in a medium; and b) recovering L-lysine from the variant or the medium in which the variant is cultured.

The culturing may be performed according to a suitable medium and culture conditions known in the art, and any person skilled in the art may easily adjust and use the medium and culture conditions. Specifically, the medium may be a liquid medium, but is not limited thereto. The culturing method may include, for example, batch culture, continuous culture, fed-batch culture, or combinations thereof, but is not limited thereto.

According to a specific embodiment of the present disclosure, the medium should meet the requirements of a specific strain in a proper manner, and may be appropriately modified by a person skilled in the art. For the culture medium for the strain of the genus *Corynebacterium,* reference may be made to a known document *(*Manual of Methods for General Bacteriology. American Society for Bacteriology. Washington D.C., USA, 1981), but is not limited thereto.

According to a specific embodiment of the present disclosure, the medium may include various carbon sources, nitrogen sources, and trace element components. Carbon sources that may be used include saccharides and carbohydrates such as glucose, sucrose, lactose, fructose, maltose, starch, cellulose, *etc.,* oils and fats such as soybean oil, sunflower oil, castor oil, coconut oil, *etc.,* fatty acids such as palmitic acid, stearic acid, and linoleic acid, alcohols such as glycerol and ethanol, and organic acids such as acetic acid. These substances may be used individually or in a mixture, but are not limited thereto. Nitrogen sources that may be used include peptone, yeast extract, meat extract, malt extract, corn steep liquor, soybean meal, urea, or inorganic compounds, e.g., ammonium sulfate, ammonium chloride, ammonium phosphate, ammonium carbonate, and ammonium nitrate. The nitrogen sources may also be used individually or in a mixture, but are not limited thereto. Phosphorus sources that may be used may include potassium dihydrogen phosphate or dipotassium hydrogen phosphate or the corresponding sodium-containing salts, but are not limited thereto. The culture medium may include metal salts such as magnesium sulfate or iron sulfate, which are required for growth, but is not limited thereto. In addition, the culture medium may include essential growth substances such as amino acids and vitamins. Moreover, suitable precursors may be used in the culture medium. The medium or individual components may be added to the culture medium batchwise or in a continuous manner by a suitable method during culturing, but are not limited thereto.

According to a specific embodiment of the present disclosure, pH of the culture medium may be adjusted by adding compounds such as ammonium hydroxide, potassium hydroxide, ammonia, phosphoric acid, and sulfuric acid to the microorganism culture medium in an appropriate manner during the culturing. In addition, during the culturing, foaming may be suppressed using an antifoaming agent such as a fatty acid polyglycol ester. Additionally, to keep the culture medium in an aerobic condition, oxygen or an oxygen-containing gas (e.g., air) may be injected into the culture medium. The temperature of the culture medium may be generally 20°C to 45°C, for example, 25°C to 40°C. The culturing may be continued until a desired amount of the useful substance is produced. For example, the culturing time may be 10 hours to 160 hours.

According to a specific embodiment of the present disclosure, in the step of recovering L-lysine from the cultured variant and the medium in which the variant is cultured, the produced L-lysine may be collected or recovered from the culture medium using a suitable method known in the art depending on the culture method. For example, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity, and size exclusion), *etc.* may be used, but the method is not limited thereto.

According to a specific embodiment of the present disclosure, in the step of recovering lysine, the culture medium is centrifuged at a low speed to remove biomass, and the obtained supernatant may be separated through ion exchange chromatography.

According to a specific embodiment of the present disclosure, the step of recovering L-lysine may include a process of purifying L-lysine.

### Advantageous Effects

*A Corynebacterium glutamicum* variant according to the present disclosure may improve a production yield of L-lysine by increasing or enhancing the expression of a gene encoding diaminopimelate decarboxylase, as compared to a parent strain.

### Brief Description of the Drawing

FIG. 1 shows a construction of a pCGI(Pm1-argS+lysA) vector including a promoter of argS-lysA operon, in which CG at positions -17 and -18 of the promoter are substituted with CT according to one exemplary embodiment of the present disclosure;
FIG. 2 shows a construction of a pCGI(Pm2-argS+lysA) vector including a promoter of argS-lysA operon, in which CG at positions -17 and -18 of the promoter are substituted with GA according to one exemplary embodiment of the present disclosure; and

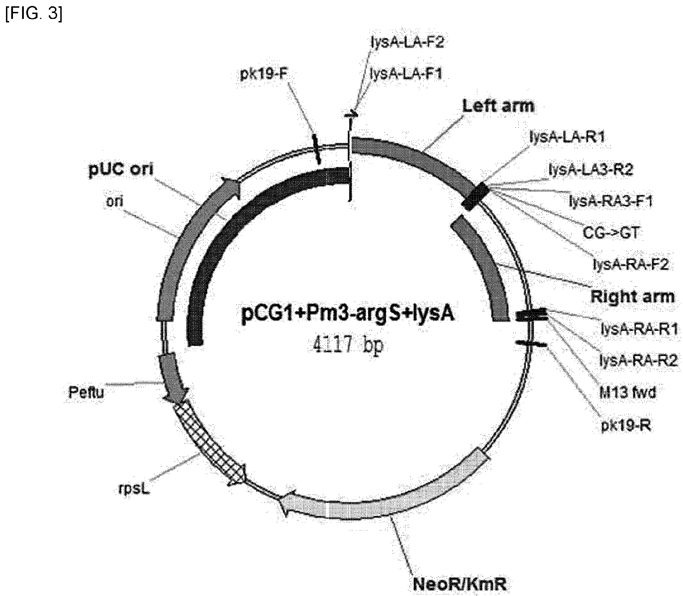

FIG. 3 shows a construction of a pCGI(Pm3-argS+lysA) vector including a promoter of argS-lysA operon, in which CG at positions -17 and -18 of the promoter are substituted with GT according to one exemplary embodiment of the present disclosure.

### Mode for Carrying Out the Invention

Hereinafter, the present disclosure will be described in more detail. However, this description is merely provided to aid understanding of the present disclosure, and the scope of the present disclosure is not limited by this exemplary description.

### Example 1. Preparation of Corynebacterium glutamicum variant

To prepare a *Corynebacterium glutamicum* variant with the enhanced diaminopimelate decarboxylase activity, *Corynebacterium glutamicum* DS1 strain and *E*. *coli* DH5a (HIT Competent cells^{™}, Cat. No. RH618) were used.

The *Corynebacterium glutamicum* DS1 strain was cultured at a temperature of 30°C in a CM-broth medium (pH 6.8) having a composition of 5 g of glucose, 2.5 g of NaCl, 5.0 g of yeast extract, 1.0 g of urea, 10.0 g of polypeptone, and 5.0 g of a beef extract in 1 L of distilled water.

The *E. coli* DH5a was cultured at a temperature of 37°C in an LB medium having a composition of 10.0 g of tryptone, 10.0 g of NaCl, and 5.0 g of a yeast extract in 1 L of distilled water.

As antibiotics, kanamycin and streptomycin, products of Sigma were used. tDNA sequencing analysis was conducted at Macrogen Co., Ltd.

### 1-1. Preparation of recombinant vector

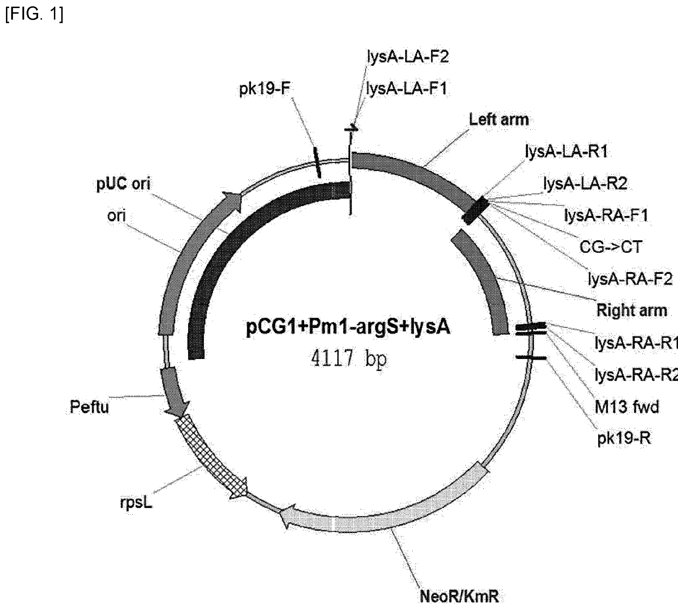

To increase lysine productivity in the strain, enhancement of diaminopimelate decarboxylase, which acts in the last step of the lysine biosynthetic pathway, was introduced. The method used in this Example induced a specific mutation in a promoter of argS-lysA operon in order to increase expression of lysA gene encoding diaminopimelate decarboxylase. The nucleotide sequence at the positions -17 and -18 of the promoter of the argS-lysA operon was replaced from CG to CT, and the 510 bp of the left arm and the 480 bp of the right arm centered around the argS-lysA operon on the genome of *Corynebacterium glutamicum* were amplified by PCR, and linked using overlap PCR, and then cloned into a recombinant vector pCGI (see Kim et al., Journal of Microbiological Methods 84 (2011) 128-130). The plasmid was named pCGI (Pm1-argS+lysA) (see FIG. 1). To construct the plasmid, primers shown in Table 1 below were used to amplify each gene fragment.

**[Table 1]**

| Primers | | | SEQ ID NO. |
|---|---|---|---|
| Primers for amplifying lysA left homology arm | lysA-LA-F1 | 5' - tgattacgcctccgcgaggctgcactgcaa - 3' | 5 |
| | lysA-LA-F2 | 5' - tccgcgaggctgcactgcaa - 3' | 6 |
| | lysA-LA-R1 | 5' - gtacacccgtcgcacagaat - 3' | 7 |
| | lysA-LA-R2 | 5' - tctagcagaggtacacccgt - 3' | 8 |
| Primers for amplifying lysA right homology arm | lysA-RA-F1 | 5' - ctctgctagaatttctccccatgacaccag - 3' | 9 |
| | lysA-RA-F2 | 5' - atttctccccatgacaccag - 3' | 10 |
| | lysA-RA-R1 | 5' - gcacacgacccaaagagtca - 3' | 11 |
| | lysA-RA-R2 | 5' - gaagcctccagcacacgacc - 3' | 12 |

PCR was performed using the above primers under the following conditions. 25 to 30 cycles were performed using a thermocycler (TP600, TAKARA BIO Inc., Japan) in the presence of 1 unit of pfu-X DNA polymerase mix (Solgent) using 1 pM of oligonucleotide and 10 ng of chromosomal DNA of *Corynebacterium glutamicum* DS1 strain as a template in a reaction solution to which 100 µM of each deoxynucleotide triphosphate (dATP, dCTP, dGTP, dTTP) was added. PCR was performed under conditions of (i) denaturation step: at 94°C for 30 seconds, (ii) annealing step: at 58°C for 30 seconds, and (iii) extension step: at 72°C for 1 minute to 2 minutes (polymerization time of 2 minutes per 1 kb).

Each gene fragment thus prepared was cloned into the pCGI vector using self-assembly cloning. The vector was transformed into *E*. *coli* DH5a, plated on an LB-agar plate containing 50 µg/mL kanamycin, and cultured at 37°C for 24 hours. The finally formed colonies were isolated, and it was confirmed whether the insert was correctly present in the vector. This vector was isolated and used in recombination of the *Corynebacterium glutamicum* strain.

As a process commonly used in the above method, the corresponding genes were amplified by PCR from genomic DNA of *Corynebacterium glutamicum* DS1 strain, and inserted into the pCGI vector using a self-assembled cloning method according to the strategy, and selected in *E. coli* DH5a. For chromosomal base substitution, each gene fragment was individually amplified, and the target DNA fragment was prepared by overlap PCR. During gene manipulation, Ex Taq polymerase (Takara) and Pfu polymerase (Solgent) were used as PCR amplification enzymes, and various restriction enzymes and DNA modifying enzymes available from NEB were used according to the supplied buffer and protocol.

### 1-2. Preparation of variant

DS3 strain which is a strain variant was prepared using the pCGI (Pm1-argS+lysA) vector. The vector was prepared at a final concentration of 1 µg/µL, and primary recombination was induced into the *Corynebacterium glutamicum* DS1 strain using electroporation (see Tauch et al., FEMS Microbiology letters 123 (1994) 343-347). The electroporated strain was then spread on a CM-agar plate containing 20 µg/µL kanamycin to isolate colonies, and then it was confirmed through PCR and base sequencing analysis whether the vector was properly inserted into the induced position on the genome. To induce secondary recombination, the isolated strain was inoculated into a CM-agar liquid medium containing streptomycin, cultured overnight or longer, and then spread on an agar medium containing the same concentration of streptomycin to isolate colonies. After examining kanamycin resistance in the finally isolated colonies, it was confirmed through base sequencing analysis whether mutations were introduced into the lysA gene in strains without antibiotic resistance (see Schafer et al., Gene 145 (1994) 69-73). Finally, *Corynebacterium glutamicum* variant (DS3) into which the mutated lysA gene was introduced was obtained.

### Example 2. Preparation of Corynebacterium glutamicum variant

*A Corynebacterium glutamicum* variant was prepared in the same manner as Example 1, except that the nucleotide sequence at the positions -17 and -18 of the promoter of the argS-lysA operon was replaced from CG to GA.

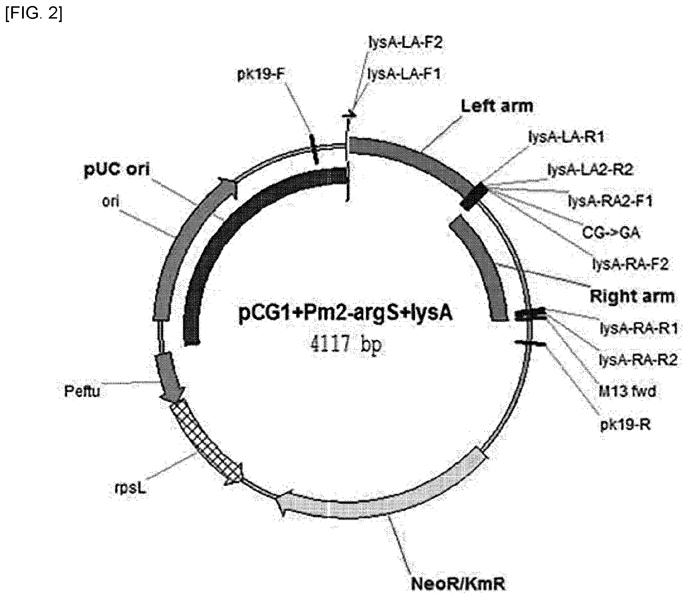

Here, to construct the plasmid, primers shown in Table 2 below were used to amplify each gene fragment, and DS3-1 strain which is a strain variant was prepared using the prepared plasmid pCGI(Pm2-argS+lysA) vector (see FIG. 2). Finally, *Corynebacterium glutamicum* variant (DS3-1) into which the mutated lysA gene was introduced was obtained.

**[Table 2]**

| Primers | | | SEQ ID NO: |
|---|---|---|---|
| Primers for amplifying lysA left homology arm | lysA-LA-F1 | 5' - tgattacgcctccgcgaggctgcactgcaa - 3' | 5 |
| | lysA-LA-F2 | 5' - tccgcgaggctgcactgcaa - 3' | 6 |
| | lysA-LA-R1 | 5' - gtacacccgtcgcacagaat - 3' | 7 |
| | lysA-LA2-R2 | 5' - tctagctcaggtacacccgt - 3' | 13 |
| Primers for amplifying lysA right homology arm | lysA-RA2-F1 | 5' - ctgagctagaatttctccccatgacaccag - 3' | 14 |
| | lysA-RA-F2 | 5' - atttctccccatgacaccag - 3' | 10 |
| | lysA-RA-R1 | 5' - gcacacgacccaaagagtca - 3' | 11 |
| | lysA-RA-R2 | 5' - gaagcctccagcacacgacc - 3' | 12 |

### Example 3. Preparation of Corynebacterium glutamicum variant

A *Corynebacterium glutamicum* variant was prepared in the same manner as Example 1, except that the nucleotide sequence at the positions -17 and -18 of the promoter of the argS-lysA operon was replaced from CG to GT.

Here, to construct the plasmid, primers shown in Table 3 below were used to amplify each gene fragment, and DS3-2 strain which is a strain variant was prepared using the prepared plasmid pCGI(Pm3-argS+lysA) vector. Finally, *Corynebacterium glutamicum* variant (DS3-2) into which the mutated lysA gene was introduced was obtained.

**[Table 3]**

| Primers | | | SEQ ID NO. |
|---|---|---|---|
| Primers for amplifying lysA left homology arm | lysA-LA-F1 | 5' - tgattacgcctccgcgaggctgcactgcaa - 3' | 5 |
| | lysA-LA-F2 | 5' - tccgcgaggctgcactgcaa - 3' | 6 |
| | lysA-LA-R1 | 5' - gtacacccgtcgcacagaat - 3' | 7 |
| | lysA-LA3-R2 | 5' - tctagcacaggtacacccgt - 3' | 15 |
| Primers for amplifying lysA right homology arm | lysA-RA3-F1 | 5' - ctgtgctagaatttctccccatgacaccag - 3' | 16 |
| | lysA-RA-F2 | 5' - atttctccccatgacaccag - 3' | 10 |
| | lysA-RA-R1 | 5' - gcacacgacccaaagagtca - 3' | 11 |
| | lysA-RA-R2 | 5' - gaagcctccagcacacgacc - 3' | 12 |

### Experimental Example 1. Comparison of L-lysine productivity between variants

The L-lysine productivity was compared between the parent strain *Corynebacterium glutamicum* DS1 strain, and DS2 strain, DS2-1 strain, and DS2-2 strain which are the lysine producing variants prepared in Examples 1 to 3.

Each strain was inoculated into a 100 mL flask containing 10 mL of a lysine medium with a composition as shown in Table 4 below, and cultured at 30°C for 48 hours with shaking at 180 rpm. After completion of the culture, lysine analysis was performed by measuring the production of L-lysine using HPLC (Shimazu, Japan), and the results are shown in Table 5.

**[Table 4]**

| Composition | Content (based on 1 L of distilled water) |
|---|---|
| Glucose | 100 g |
| Ammonium sulfate | 55 g |
| (NH₄)₂SO₄ | 35 g |
| KH₂PO₄ | 1.1 g |
| MgSO₄·H₂O | 1.2 g |
| MnSO₄·H₂O | 180 mg |
| FeSO₄·H₂O | 180 mg |
| Thiamine·HCl | 9 mg |
| Biotin | 1.8 mg |
| CaCO₃ | 5% |
| pH | 7.0 |

**[Table 5]**

| Strain | OD610 | L-Lysine (g/L) | L-Lysine productivity |
|---|---|---|---|
| | | | (g/gDCW) |
| Parent strain (DS1) | 24.0 | 64.8 | 6.4 |
| Strain variant (DS3) | 23.0 | 65.2 | 6.7 |
| Strain variant (DS3-1) | 23.0 | 65.8 | 6.8 |
| Strain variant (DS3-2) | 22.0 | 66.9 | 7.2 |

As shown in Table 5, *Corynebacterium glutamicum* variants DS3, DS3-1, and DS3-2 exhibited about 4.7%, 6.3%, and 12.5% increases in the L-lysine productivity, respectively, as compared to the parent strain *Corynebacterium glutamicum* DS1 strain, due to substitution of the specific positions (-17 and -18 regions) in the promoter sequence of the argS-lysA operon with the optimal nucleotide sequence (CT, GA or GT) for strengthening the lysine biosynthetic pathway. These results indicate that enhanced expression of the lysA gene improved the L-lysine producing ability of the strain by promoting the decomposition of the carbon bonds of the lysine precursor.

## Claims

1. A promoter in which a nucleotide sequence at positions 183 and 184 in a promoter sequence of SEQ ID NO: 1 of a gene encoding diaminopimelate decarboxylase is substituted from CG to GT.

2. A *Corynebacterium glutamicum* variant with improved L-lysine producing ability, wherein a nucleotide sequence at positions 183 and 184 in a promoter sequence of SEQ ID NO: 1 of a gene encoding diaminopimelate decarboxylase is substituted from CG to GT.

3. The *Corynebacterium glutamicum* variant of claim 2, wherein the variant comprises a nucleotide sequence represented by SEQ ID NO: 4.

4. A method of producing L-lysine, the method comprising:
a) culturing the variant of claim 2 in a medium; and
b) recovering L-lysine from the variant or the medium in which the variant is cultured.

## Patentansprüche

1. Promotor, bei dem eine Nukleotidsequenz an den Positionen 183 und 184 in einer Promotorsequenz von SEQ ID NO: 1 eines Gens, das für Diaminopimelat-Decarboxylase kodiert, von CG durch GT substituiert ist.

2. *Corynebacterium glutamicum-Stamm* mit verbesserter L-Lysin-Produktionsfähigkeit, wobei eine Nukleotidsequenz an den Positionen 183 und 184 in einer Promotorsequenz von SEQ ID NO: 1 eines Gens, das für Diaminopimelat-Decarboxylase kodiert, von CG durch GT substituiert ist.

3. *Corynebacterium glutamicum-Stamm* nach Anspruch 2, wobei der Stamm eine Nukleotidsequenz umfasst, die durch SEQ ID NO: 4 dargestellt ist.

4. Verfahren zur Herstellung von L-Lysin, wobei das Verfahren Folgendes umfasst:
a) Kultivieren des Stamms nach Anspruch 2 in einem Medium; und
b) Gewinnen von L-Lysin aus dem Stamm oder dem Medium, in dem der Stamm kultiviert wird.

## Revendications

1. Promoteur dans lequel une séquence nucléotidique aux positions 183 et 184 d'une séquence promotrice de SEQ ID NO: 1 d'un gène codant pour la diaminopimélate décarboxylase est substituée de CG à GT.

2. Variant *de Corynebacterium glutamicum* présentant une capacité améliorée de production de L-lysine, dans laquel une séquence nucléotidique aux positions 183 et 184 d'une séquence promotrice de SEQ ID NO: 1 d'un gène codant pour la diaminopimélate décarboxylase est substituée de CG à GT.

3. Variant de *Corynebacterium glutamicum* selon la revendication 2, dans laquel le variant comprend une séquence nucléotidique représentée par SEQ ID NO: 4.

4. Procédé de production de L-lysine, le procédé comprenant :
a) la culture du variant selon la revendication 2 dans un milieu ; et
b) la récupération de la L-lysine à partir du variant ou du milieu dans lequel la variant est cultivé.
